# EUROPEAN PATENT APPLICATION

(11) **EP 2 992 804 A1**
(43) Date of publication of application: **09.03.2016**
(21) Application number: 14885826.9
(22) Date of filing: 04.11.2014
(51) Int. Cl.: A61B 1/00, G02B 23/24

(54) **ENDOSCOPE LEAK TEST DEVICE**

(30) Priority: 11.03.2014 JP 2014047921
(71) Applicant: Olympus Corporation, Shibuya-ku Tokyo 151-0072 (JP)
(72) Inventor: YOSHIE Michifumi, Tokyo 151-0072 (JP)
(74) Representative: Gunzelmann, Rainer
(86) International application number: PCT/JP2014/079253
(87) International publication number: WO 2015/136767

(57) **Abstract**

An endoscope leak inspection apparatus includes an endoscope connecting section communicably connected to an inside of an endoscope, a gas feed section that communicates with the endoscope connecting section and feeds gas, a measuring section that measures an internal pressure of the endoscope, an endoscope-information recognizing section that recognizes serial numbers uniquely allocated to individual endoscopes, a storing section that stores the serial numbers and measurement results of the measuring section in association with each other, a threshold setting section that sets a threshold on the basis of measurement results in past having the same serial number stored in the storing section, and a leak determining section that compares the threshold and a measurement result by the measuring section and determines presence or absence of a leak of the endoscope.

## Description

### Technical Field

The present invention relates to an endoscope leak inspection apparatus that feeds gas to an inside of an endoscope, measures pressure, and performs inspection of a leak.

### Background Art

In general, at least a part of respective components configuring an endoscope are disposed in an airtightness secured region for a purpose of preventing function deterioration or performance deterioration due to a factor such as intrusion of liquid.

In a general endoscope, a problem could occur that, for example, because of mishandling of the endoscope including a constituent member such as a rubber member used for forming an airtightness secured region, the airtightness of the region is deteriorated. Therefore, for example, before the endoscope is used or before the used endoscope is cleaned, a leak inspection for checking whether the airtightness of the endoscope is secured is conventionally performed. For example, Japanese Patent Application Laid-Open Publication No. 2001-245839 discloses a leak detection apparatus usable for the leak inspection.

More specifically, Japanese Patent Application Laid-Open Publication No. 2001-245839 discloses a leak detection apparatus used for an endoscope including a waterproof structure portion, the leak detection apparatus being a configuration for determining presence or absence of a hole, a crack, or the like in the waterproof structure portion on the basis of a comparison result of pressure detected after an end of gas feed to the waterproof structure portion and pressure detected continuously for a predetermined time after the end of the gas feed to the waterproof structure portion.

However, according to the configuration disclosed in Japanese Patent Application Laid-Open Publication No. 2001-245839, presence or absence of an abnormal state in the waterproof structure portion is determined without taking into account a capacity change of an endoscope inside involved in aged deterioration of constituent members.

The present invention has been devised in view of the circumstances and it is an object of the present invention to provide an endoscope leak inspection apparatus capable of checking, taking into account aged deterioration of an endoscope, whether airtightness of the endoscope is secured.

### Disclosure of Invention

### Means for Solving the Problem

An endoscope leak inspection apparatus in an aspect of the present invention includes: an endoscope connecting section communicably connected to an inside of an endoscope; a gas feed section that communicates with the endoscope connecting section and feeds gas; a measuring section that measures an internal pressure of the endoscope; an endoscope-information recognizing section that recognizes serial numbers uniquely allocated to individual endoscopes; a storing section that stores the serial numbers and measurement results of the measuring section in association with each other; a threshold setting section that sets a threshold on the basis of measurement results in past having the same serial number stored in the storing section; and a leak determining section that compares the threshold and a measurement result by the measuring section and determines presence or absence of a leak of the endoscope.

### Brief Description of the Drawings

Fig. 1 is a diagram showing a configuration of a main part of an inspection system including an endoscope leak inspection apparatus according to an embodiment;
Fig. 2 is a diagram for explaining an example of a configuration of a main body device included in the endoscope leak inspection apparatus according to the embodiment;
Fig. 3 is a flowchart for explaining an example of processing and the like performed by the endoscope leak inspection apparatus according to the embodiment; and
Fig. 4 is a diagram showing an example of changes with time of thresholds TH1 and TH2 set by the processing shown in Fig. 3.

### Best mode for Carrying Out the Invention

An embodiment of the present invention is explained below with reference to the drawings.

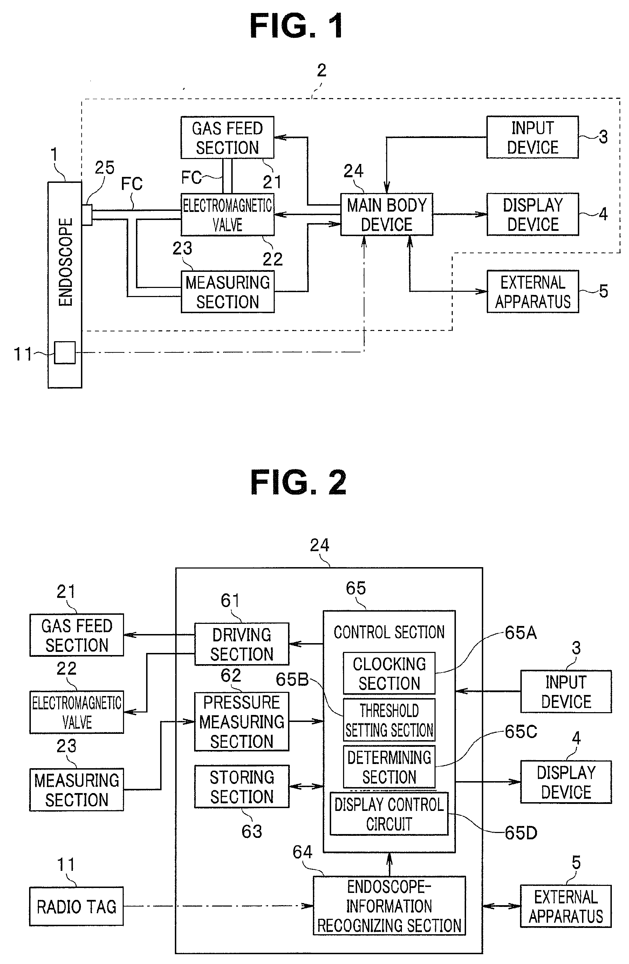

Fig. 1 to Fig. 4 are diagrams related to the embodiment of the present invention.

Fig. 1 is a diagram showing a configuration of a main part of an inspection system including an endoscope leak inspection apparatus according to the embodiment. As shown in Fig. 1, a flexible endoscope 1 is connected to an endoscope leak inspection apparatus 2 of the present invention. The leak inspection apparatus 2 may include an input device 3 and a display device 4. Alternatively, the input device 3 and the display device 4 may be configured to be externally connectable. Further, the endoscope leak inspection apparatus 2 of the present invention can also be used together with an external apparatus 5 connected to a main body device 24 of the endoscope leak inspection apparatus 2 via a communication line (not shown in the figure). Details of the external apparatus 5 are explained below.

On an inside of the endoscope 1, for example, one or more airtight regions with airtightness secured by rubber members or the like are provided. The endoscope 1 includes a radio tag 11 configured by RFID (radio frequency identification) or the like capable of transmitting, by radio, endoscope information including identification information such as serial numbers individually allocated to individual endoscopes 1 to the main body device 24 of the endoscope leak inspection apparatus 2.

The endoscope leak inspection apparatus 2 includes, for example, as shown in Fig. 1, a gas feed section 21, an electromagnetic valve 22, a measuring section 23, and a main body device 24.

Specific examples of the gas feed section 21 include an air pump. The gas feed section 21 is communicably connected to the endoscope 1 via a gas channel FC and an endoscope connecting section 25 provided at a distal end of the gas channel FC, for example. The gas feed section 21 is configured to perform or stop, on the basis of a driving signal outputted from the main body device 24, supply of air for pressurizing the inside of the endoscope 1. Note that the endoscope connecting section 25 may be directly connected to the endoscope or may be connected to the endoscope via a tube (not shown in the figure) externally attached between the endoscope connecting section 25 and the endoscope.

The electromagnetic valve 22 is provided in a predetermined position in the gas channel FC between the endoscope 1 and the gas feed section 21. The electromagnetic valve 22 is configured to perform, on the basis of a driving signal outputted from the main body device 24, an opening and closing operation for changing to an open state or a closed state.

In the gas channel FC, the measuring section 23 is disposed between the endoscope connecting section 25 and the electromagnetic valve 22. The measuring section 23 is configured to detect pressure of the gas channel FC sandwiched by the endoscope connecting section 25 and the electromagnetic valve 22 in a case in which the electromagnetic valve 22 is in the closed state and generate an electric signal corresponding to the detected pressure and output the electric signal to the main body device 24. The gas channel FC sandwiched by the endoscope connecting section 25 and the electromagnetic valve 22 communicates with the endoscope inside. Therefore, an internal pressure of the gas channel FC can be read as an internal pressure of the endoscope.

The main body device 24 includes, for example, as shown in Fig. 2, a driving section 61, a pressure measuring section 62, a storing section 63, an endoscope-information recognizing section 64, and a control section 65. Fig. 2 is a diagram for explaining an example of a configuration of the main body device included in the endoscope leak inspection apparatus according to the embodiment.

The driving section 61 is configured to generate and output, on the basis of control by the control section 65, driving signals for respectively driving the gas feed section 21 and the electromagnetic valve 22.

The pressure measuring section 62 is configured to acquire a pressure value corresponding to an electric signal outputted from the measuring section 23.

The storing section 63 includes, for example, a nonvolatile storage medium and is configured to be capable of storing various kinds of information such as an inspection history including one or more kinds of inspection information related to a leak inspection for checking whether airtightness of the endoscope 1 is secured.

The endoscope-information recognizing section 64 includes, for example, an RFID reader and is configured to receive a radio signal transmitted from the radio tag 11 and output endoscope information included in the received radio signal to the control section 65. The endoscope-information recognizing section 64 includes, for example, a LAN (local area network) adapter and is configured to perform various operations related to communication between the control section 65 and the external apparatus 5. However, in the present invention, a retaining form of the endoscope information is not limited to a form of emitting a signal like the radio tag 11 and may be a form for not emitting a signal such as enumeration of numbers or signs or a barcode. In this case, the endoscope-information recognizing section 64 functions as a barcode reader for reading the barcode.

The control section 65 includes, for example, a CPU and is configured to perform an operation according to control for the driving section 61 and operation of the input device 3. The control section 65 includes, for example, a display control circuit 65D and is configured to be capable of generating a GUI (graphical user interface) such as an input screen for urging an input of identification information capable of individually identifying the inspection target endoscope 1 for which the leak inspection is performed and capable of causing the display device 4 to display the GUI. As the identification information capable of identifying the endoscopes, serial numbers different for each one endoscope can be illustrated. The control section 65 acquires both of the endoscope information inputted through the endoscope-information recognizing section 64 and a serial number of the endoscope 1 based on any one kind of information inputted on the input screen or only the serial number. The control section 65 is configured to identify a model of the endoscope 1 corresponding to the acquired endoscope information or the acquired serial number and check presence or absence of inspection information corresponding to the acquired endoscope information or the acquired serial number.

On the other hand, the control section 65 includes a clocking section 65A, a threshold setting section 65B, and a determining section 65C.

The clocking section 65A includes, for example, a real-time clock and is configured to be capable of acquiring information concerning present date and time. The clocking section 65A includes, for example, a timer and is configured to be capable of performing a clocking operation for clocking times T1 and T2 explained below.

The threshold setting section 65B is configured to set, when a check result indicating that inspection information corresponding to the serial number of the endoscope 1 is included in an inspection history is obtained, on the basis of the model of the endoscope 1 and the inspection information, a threshold used in a leak inspection for the endoscope 1. The threshold setting section 65B is configured to set, when a check result indicating that inspection information corresponding to the serial number of the endoscope 1 is not included in the inspection history is obtained, according to the model of the endoscope 1, the threshold used in the leak inspection for the endoscope 1. Note that details of processing related to the setting of the threshold are explained below.

The threshold setting section 65B is configured to set, when inspection information corresponding to the serial number included in the endoscope information inputted through the endoscope-information recognizing section 64 cannot be acquired (from the inspection history) because, for example, the leak inspection is not performed yet, the threshold used in the leak inspection for the endoscope 1 to a predetermined value corresponding to the model of the endoscope 1 corresponding to the serial number.

The determining section 65C is configured to perform determination based on a pressure fluctuation value acquired by the leak inspection and one or more thresholds set by the threshold setting section 65B to thereby perform determination related to whether the airtightness of the endoscope 1 is secured.

The input device 3 includes, for example, a keyboard, a pointing device, and the like and is configured to be capable of performing operation related to an input of information and an instruction to the control section 65.

The display device 4 includes, for example, a liquid crystal display and is configured to be capable of displaying the GUI or the like generated by the control section 65.

The external apparatus 5 is configured by, for example, another endoscope leak inspection apparatus (hereinafter referred to as endoscope leak inspection apparatus 2i) included in an inspection system different from the inspection system shown in Fig. 1. Note that the external apparatus 5 is not limited to the endoscope leak inspection apparatus 2i and may be, for example, a database server accessible from the endoscope leak inspection apparatuses 2 and 2i and configured to accumulate and retain, as a database, inspection information acquired by the endoscope leak inspection apparatuses 2 and 2i.

The control section 65 included in the main body device 24 includes, for example, an information processing apparatus such as a computer and is configured to be capable of performing communication with the control section 65 provided in the endoscope leak inspection apparatus 2i. The control section 65 may be configured to acquire, for example, inspection information of a leak inspection performed in the leak inspection apparatus 2 and inspection information of a leak inspection performed in the endoscope leak inspection apparatus 2i from the endoscope leak inspection apparatus 2i via a communication line (not shown in the figure) and store an inspection history including the acquired respective kinds of inspection information in the storing section 63. The control section 65 may be configured to be capable of performing, when a request from the main body device 24 provided in the endoscope leak inspection apparatus 2i is received, processing for extracting respective kinds of inspection information according to the request out of the inspection history stored in the storing section 63 and performing an operation for returning information related to an extraction result to the main body device 24 provided in the endoscope leak inspection apparatus 2i.

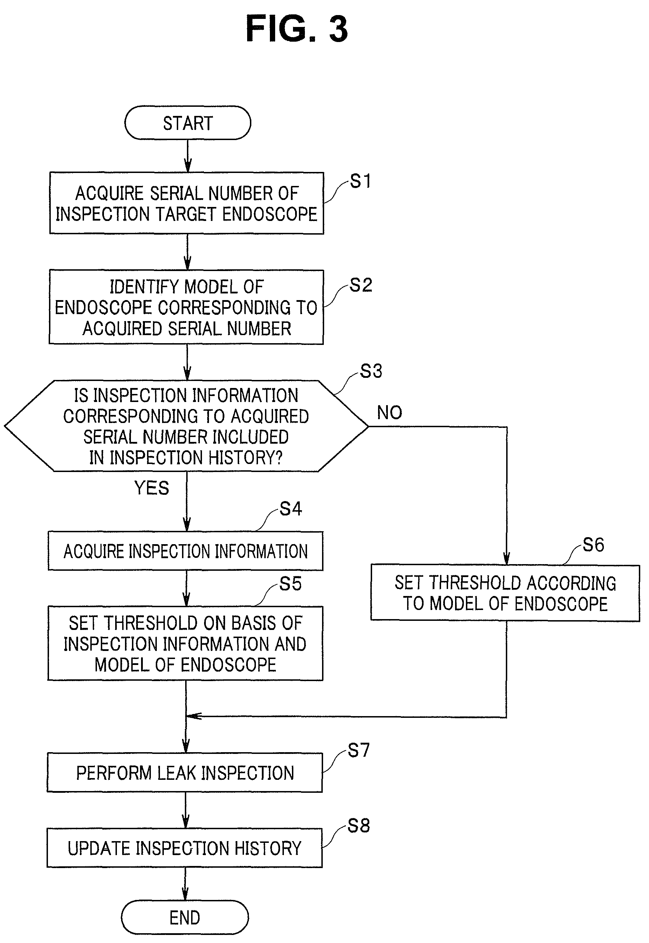

Next, action of the present embodiment is explained. First, an example of a leak inspection (a leak inspection performed in step S7 of Fig. 3 explained below) performed to obtain inspection information stored in the storing section 63 is explained.

In a state in which the endoscope 1 and the leak inspection apparatus 2 are connected via the gas channel FC, the control section 65 applies, to the driving section 61, control for changing the electromagnetic valve 22 to the open state and performing gas feed from the gas feed section 21 to the endoscope 1.

The control section 65 causes the clocking section 65A to start counting of time when the gas feed by the gas feed section 21 is started.

When the time T1 elapses after the counting by the clocking section 65A is started, the control section 65 applies, to the driving section 61, control for stopping the gas feed from the gas feed section 21 to the endoscope 1 and shifting the electromagnetic valve 22 from the open state to the closed state.

The control section 65 performs a predetermined arithmetic operation using a pressure value PA at timing TA when the time T1 elapses and a pressure value PB at timing TB when the time T2 further elapses from the timing TA to thereby calculate a pressure fluctuation value ΔP. Concerning the predetermined arithmetic operation, the control section 65 may simply calculate a difference value between PB and PA or may perform an arithmetic operation incorporating an endoscope temperature or an air temperature in order to take into account a coefficient of expansion of air.

Concerning a brand-new endoscope or an endoscope used first after repairing, the determining section 65C of the control section 65 determines, from the pressure fluctuation value ΔP calculated as explained above and a predetermined threshold peculiar to a model, whether airtightness of the endoscope 1 is secured. The predetermined threshold peculiar to the model may be owned by the determining section in advance, may be externally input, or may be acquired from an outside of the apparatus using a communication line.

Concerning an endoscope after a plurality of times of use, in order to add account aged deterioration of the endoscope to determination conditions, the determining section 65C of the control section 65 performs determination based on the pressure fluctuation value ΔP and the thresholds TH1 and TH2 set through processing in step S5 or step S6 of Fig. 3 explained below to thereby determine whether the airtightness of the endoscope 1 is secured.

More specifically, for example, when the pressure fluctuation value ΔP calculated as explained above is within a range of a value equal to or larger than the threshold TH1 and equal to or smaller than the threshold TH2 (TH1≤ΔP≤TH2 is satisfied), the determining section 65C determines that the airtightness of the endoscope 1 is secured.

For example, when the pressure fluctuation value ΔP calculated as explained above is larger than the threshold TH2 (TH2<ΔP is satisfied), the determining section 65C of the control section 65 determines that the airtightness of the endoscope 1 is not secured.

That is, with the leak inspection explained above, the determination result by the determining section 65C and the pressure fluctuation value ΔP used in obtaining the determination result are acquired as an inspection result of the leak inspection of the endoscope 1. According to the present embodiment, the storing section 63 stores an inspection history including one or more kinds of inspection information that associates the serial number of the endoscope 1 for which the leak inspection explained above is performed, an inspection result of the leak inspection corresponding to the serial number, and inspection date and time corresponding to date and time when the inspection result is obtained.

Note that the determining section 65C in the present embodiment is not limited to perform threshold determination using the pressure fluctuation value ΔP obtained by subtracting the pressure value PB from the pressure value PA to thereby determine whether the airtightness of the endoscope 1 is secured. For example, the determining section 65C may calculate a leak amount LL (mL/min) using the pressure fluctuation value ΔP and perform threshold determination using the calculated leak amount LL to thereby determine whether the airtightness of the endoscope 1 is secured. Note that, when the threshold determination is performed using the leak amount LL, in step S5 of Fig. 3 explained below, the threshold TH1 and the threshold TH2 corresponding to a unit of the leak amount LL are respectively set (by the threshold setting section 65B).

Subsequently, processing and the like in a case in which the leak inspection explained above is performed (in step S7 of Fig. 3 explained below) are explained with reference to a flowchart of Fig. 3. Fig. 3 is a flowchart for explaining an example of processing and the like performed by the endoscope leak inspection apparatus according to the embodiment.

After connecting the respective sections of the inspection system 101 as shown in Fig. 1 and turning on power supplies of the respective sections, for example, the user depresses an inspection start switch (not shown in the figure) provided in the input device 3 to thereby give an instruction for starting the leak inspection to the control section 65. Alternatively, the control section 65 determines that the acquisition of the serial number shown in step S1 is a sign of an inspection start.

In step S1 of Fig. 3, when detecting that the instruction for starting the leak inspection is performed in the input device 3, the control section 65 including a function of an identification-information acquiring section performs processing for acquiring a serial number of the inspection target endoscope 1 for which the leak inspection is performed.

More specifically, in step S1 of Fig. 3, for example, when a part where endoscope information is retained in the endoscope 1 and the endoscope-information recognizing section 64 of the leak inspection apparatus 2 are disposed within an endoscope information acquirable distance by a hand of the user, the control section 65 performs, on the basis of endoscope information inputted through the endoscope-information recognizing section 64, processing for extracting a serial number included in the endoscope information.

Alternatively, for example, the control section 65 generates an input screen for urging a manual input of a serial number of the endoscope 1 and causes the display device 4 to display the input screen and performs processing for acquiring a serial number inputted on the input screen.

In step S2 of Fig. 3, for example, the control section 65 refers to table data stored in the storing section 63 in a state in which serial numbers and model names are associated for each of respective endoscopes to thereby identify a model of the endoscope 1 corresponding to the serial number acquired by the processing in step S1 of Fig. 3.

Note that the control section 65 in the present embodiment is not limited to perform the processing for identifying a model of the endoscope 1 in step S2 of Fig. 3. For example, the control section 65 may refer to the table data stored in the storing section 63 to thereby perform processing for identifying at least one of a size of a diameter and length of an insertion section of the endoscope 1 including a shape insertable into a subject.

Thereafter, in step S3 of Fig. 3, the control section 65 performs, on the basis of the serial number acquired by the processing in step S1 of Fig. 3, processing for checking whether inspection information corresponding to the acquired serial number is included in at least one of the inspection history stored in the storing section 63 and the inspection history stored in the external apparatus 5.

More specifically, for example, the control section 65 checks whether inspection information corresponding to the serial number acquired by the processing in step S 1 of Fig. 3 is present in the inspection history stored in the storing section 63. For example, when detecting that the inspection information corresponding to the serial number acquired by the processing in step S 1 of Fig. 3 is not included in the inspection history of the storing section 63, the control section 65 requests the external apparatus 5 to return information related to an extraction result of the inspection information corresponding to the serial number. The control section 65 checks whether the inspection information corresponding to the serial number acquired by the processing in step S 1 of Fig. 3 is included in the information related to the extraction result received from the external apparatus 5.

In step S4 of Fig. 3, when obtaining a check result indicating that the inspection information corresponding to the serial number acquired by the processing in step S1 of Fig. 3 is included in the inspection history, the control section 65 including a function of an inspection-information acquiring section acquires respective kinds of inspection information corresponding to the check result from the storing section 63 or the external apparatus 5.

In step S5 of Fig. 3, the threshold setting section 65B of the control section 65 performs, on the basis of the respective kinds of inspection information acquired by the processing in step S4 of Fig. 3 and the model of the endoscope 1 identified by the processing in step S2 of Fig. 3, processing for setting one or more thresholds used in the leak inspection of strep S7 in Fig. 3.

More specifically, in step S5 of Fig. 3, for example, the threshold setting section 65B extracts, out of the respective kinds of inspection information acquired by the processing in step S4 of Fig. 3, most recent one pressure fluctuation value ΔP1 acquired when an inspection result indicating that airtightness is secured is obtained and performs an arithmetic operation for subtracting a pressure value PL of 1 pascal or more determined in advance according to the model of the endoscope 1 from the extracted pressure fluctuation value ΔP1 to thereby set the threshold TH1 (equivalent to a lower limit of a pressure value used in determination by the determining section 65C) and performs an arithmetic operation for adding the pressure value PH of 1 pascal or more determined in advance according to the model of the endoscope 1 to the extracted pressure fluctuation value ΔP1 to thereby set the threshold TH2 (equivalent to an upper limit of the pressure value used in the determination by the determining section 65C).

Alternatively, in step S5 of Fig. 3, for example, the threshold setting section 65B extracts, out of the respective kinds of inspection information acquired by the processing in step S4 of Fig. 3, the most recent one pressure fluctuation value ΔP1 acquired when the inspection result indicating that airtightness is secured is obtained and performs an arithmetic operation for multiplying the extracted pressure fluctuation value ΔP1 with a coefficient K1, which is a value smaller than 1, determined in advance according to the model of the endoscope 1 to thereby set the threshold TH1 (equivalent to the lower limit of the pressure value used in the determination by the determining section 65C) and performs an arithmetic operation for multiplying the extracted pressure fluctuation value ΔP1 with a coefficient K2, which is larger than 1, determined in advanced according to the model of the endoscope 1 to thereby set the threshold TH2 (equivalent to the upper limit of the pressure value used in the determination by the determining section 65C).

Note that, according to the present embodiment, the pressure value PL, the pressure value PH, the coefficient K1, and the coefficient K2 are not limited to be set as the values determined in advance according to the model of the endoscope 1 and may be set as, for example, values according to operation of the input device 3.

For example, when setting the threshold TH1 and the threshold TH2 in step S5 of Fig. 3, the threshold setting section 65B in the present embodiment may apply, to at least one of the coefficient K1 and the coefficient K2, weighting corresponding to an elapsed period from acquisition date and time of the pressure fluctuation value ΔP1 to present date and time (date and time when a leak inspection for the endoscope 1 is performed).

More specifically, for example, when the elapsed period from the acquisition date and time of the pressure fluctuation value ΔP1 to the present date and time is equal to or longer than a period TP1, the threshold setting section 65B may apply, to at least one of the coefficient K1 and the coefficient K2, weighting for moving a division value DV1 obtained by dividing the coefficient K1 by the coefficient K2 (=K1/K2) away from 1 (moving the division value DV1 close to 0). On the other hand, for example, when the elapsed period from the acquisition date and time of the pressure fluctuation value ΔP1 to the present date and time is equal to or shorter than a period TP2 sufficiently shorter than the period TP1, the threshold setting section 65B may apply, to at least one of the coefficient K1 and the coefficient K2, weighting for moving a division value DV2 obtained by dividing the coefficient K2 by the coefficient K1 (=K2/K1) away from 1 (moving the division value DV2 close to 0).

In step S5 of Fig. 3, the threshold setting section 65B in the present embodiment is not limited to set the thresholds TH1 and TH2 using the most recent one pressure fluctuation value ΔP1 acquired when the inspection result indicating that airtightness is secured is obtained. For example, the threshold setting section 65B may extract, out of the respective kinds of inspection information acquired by the processing in step S4 of Fig. 3, a most recent plurality of pressure fluctuation values ΔP1 to ΔPK (2≤k) acquired when the inspection result indicating that airtightness is secured is obtained, calculate an average AVR of the extracted plurality of pressure fluctuation values ΔP1 to ΔPk, and set the thresholds TH1 and TH2 using the calculated average AVR.

On the other hand, when obtaining, in step S3 of Fig. 3, a check result indicating that the inspection information corresponding to the serial number acquired by the processing in step S 1 of Fig. 3 is not included in the inspection history is obtained, in step S6 of Fig. 3, the threshold setting section 65B respectively sets the thresholds TH1 and TH2 to be predetermined values corresponding to the model of the endoscope 1 identified by the processing in step S2 of Fig. 3.

The control section 65 performs, in step S7 of Fig. 3, the leak inspection explained above using the thresholds TH1 and TH2 set through the processing in step S5 or step S6 of Fig. 3 to thereby acquire, as an inspection result, the determination result by the determining section 65C and the pressure fluctuation value ΔP1 used in obtaining the determination result.

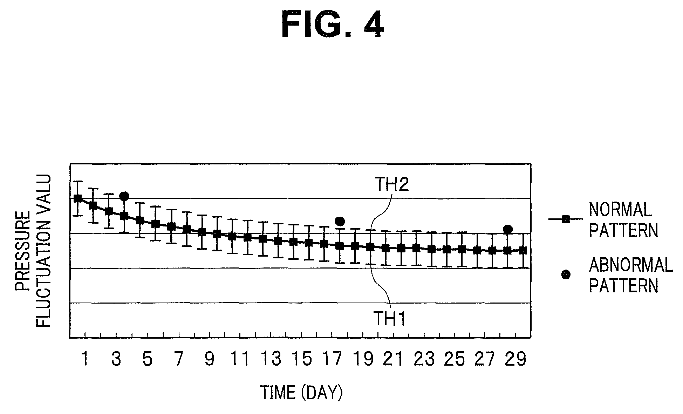

The control section 65 associates the serial number acquired by the processing in step S1 of Fig. 3, the inspection result acquired by the leak inspection in step S7 of Fig. 3, and the inspection date and time equivalent to the date and time when the inspection result is obtained to thereby generate inspection information. In step S8 of Fig. 3, after updating the inspection history stored in the storing section 63 with the inspection information generated as explained above, the control section 65 completes a series of processing. The series of processing shown in Fig. 3 is repeatedly performed, whereby the thresholds TH1 and TH2 change with time, for example, as shown in Fig. 4. Fig. 4 is a diagram showing an example of the change with time of the thresholds TH1 and TH2 set by the processing shown in Fig. 3.

As explained above, according to the present embodiment, it is possible to set, on the basis of inspection information of a predetermined leak inspection performed in the past for one endoscope, thresholds suitable for the predetermined leak inspection currently performed for the one endoscope. As a result, according to the present embodiment, it is possible to prevent occurrence of misdetection in inspection for checking whether airtightness of the endoscope is secured.

On the other hand, according to the present embodiment, for example, the endoscope leak inspection apparatus 2 of the present invention may be built in endoscope cleaning/disinfecting apparatuses disclosed in Japanese Patent Application Laid-Open Publication No. 2009-226193 and Japanese Patent Application Laid-Open Publication No. 2010-035936.

More specifically, the leak inspection apparatus 2 of the present invention may be included in an endoscope cleaning/disinfecting apparatus including a cleaning/disinfecting tank configured to be capable of storing cleaning liquid used for cleaning of an endoscope, a cleaning-liquid supply section configured to supply the cleaning liquid used for the cleaning of the inspected endoscope to the cleaning/disinfecting tank, and a cleaning-liquid discharge section configured to discharge the cleaning liquid used for the cleaning of the inspected endoscope from the cleaning/disinfecting tank.

When Japanese Patent Application Laid-Open Publication No. 2009-226193 is referred to as an example, a compressor 22 described in the publication can be used as the gas feed section 21 of the present invention. An RFID reader 62 described in the publication can be used as the endoscope-information recognizing section 64 of the present invention. An RFID tag 61 described in the publication corresponds to the radio tag 11 of the present invention.

When Japanese Patent Application Laid-Open Publication No. 2010-35936 is referred to as an example, a pressure sensor 77b described in the publication can be used as the measuring section 23 of the present invention. A scope recognizing section 75 described in the publication can be used as the endoscope-information recognizing section 64 of the present invention. A control section 70 described in the publication can be used as the control section 65 of the present invention. A display section 76 described in the publication can be used as the display device 4 of the present invention.

Note that the present invention is not limited to the embodiment explained above. Naturally, various changes and applications are possible within a range not departing from the spirit of the invention.

This application is based upon and claims the benefit of priority from Japanese Patent Application No. 2014-047921 filed in Japan on March 11, 2014, the disclosed contents of which are incorporated in the specification, the claims, and the drawings of this application.

## Claims

1. An endoscope leak inspection apparatus comprising:
an endoscope connecting section communicably connected to an inside of an endoscope;
a gas feed section that communicates with the endoscope connecting section and feeds gas;
a measuring section that measures an internal pressure of the endoscope;
an endoscope-information recognizing section that recognizes serial numbers uniquely allocated to individual endoscopes;
a storing section that stores the serial numbers and measurement results of the measuring section in association with each other;
a threshold setting section that sets a threshold on the basis of measurement results in past having a same serial number stored in the storing section; and
that compares the threshold and a measurement result by the measuring section and determines presence or absence of a leak of the endoscope.

2. The endoscope leak inspection apparatus according to claim 1, wherein the threshold setting section sets, as an upper limit threshold, a value obtained by adding a first value to a latest value among the measurement results in the past having the same serial number stored in the storing section and sets, as a lower limit threshold, a value obtained by subtracting a second value from the latest value, and
when the measurement result by the measuring section is not within a range of the upper limit threshold to the lower limit threshold, the leak determining section determines that there is a leak place in the endoscope.

3. The endoscope leak inspection apparatus according to claim 1 or 2, wherein the storing section stores, in association with the measurement result, a date and time when the measurement is performed.

4. The endoscope leak inspection apparatus according to claim 3, wherein
when a measurement date and time of the internal pressure measured latest is before a predetermined period, the threshold setting section sets, as an upper limit threshold, a value obtained by adding a fourth value to a value obtained by subtracting a third value from the latest internal pressure and sets, as a lower limit threshold, a value obtained by subtracting a fifth value from the value.

5. The endoscope leak inspection apparatus according to claim 3 or 4, further comprising:
a threshold-information transmitting section that transmits the threshold of the endoscope associated with the serial number to an outside as threshold information; and
a threshold-information receiving section that receives the threshold information transmitted from the outside, wherein
the leak determining section compares latest one of the threshold set by the threshold setting section and the threshold information received by the threshold-information receiving section and the measurement result by the measuring section and determines presence or absence of a leak of the endoscope.

6. The endoscope leak inspection apparatus according to any one of claims 1 to 4, further comprising an internal-pressure-information receiving section that receives internal pressure information of the endoscope transmitted from an outside, the internal pressure information being associated with the serial numbers uniquely allocated to the individual endoscopes, wherein
the storing section stores the internal pressure information received by the threshold-information receiving section, and
the threshold setting section sets the threshold on the basis of the internal pressure information received by the internal-pressure receiving section.
